# EUROPEAN PATENT SPECIFICATION

(11) **EP 3 556 312 B1**
(45) Date of publication and mention of the grant of the patent: **16.11.2022**
(21) Application number: 19169669.9
(22) Date of filing: 16.04.2019
(51) Int. Cl.: A61B 34/20, A61B 90/00, A61B 5/06

(54) **A THREE-DIMENSIONAL VISION SYSTEM**
EIN DREIDIMENSIONALES VISIONSYSTEM
UN SYSTÈME DE VISION TRIDIMENSIONNEL

(30) Priority: 16.04.2018 IT 201800004552
(43) Date of publication of application: 23.10.2019
(73) Proprietor: MASMEC S.p.A., 70026 Modugno (IT)
(72) Inventor: LARIZZA, Pietro, 70026 Modugno (BA) (IT)
(74) Representative: Bongiovanni, Simone

(56) References cited:
- EP-A2- 1 096 268
- WO-A2-2011/128766
- WO-A2-2017/208186
- US-A1- 2006 058 604
- US-A1- 2017 069 073
- US-B2- 7 567 833

## Description

### TECHNICAL FIELD OF THE INVENTION

The present invention relates to a three-dimensional vision system for guiding a surgical/diagnostic instrument in the body of a patient.

### BACKGROUND ART

As is known, nowadays many surgical and/or diagnostic procedures are performed using a surgical/diagnostic instrument ("operating tool") that can be inserted percutaneously to reach an area affected by disease (often referred to as the target zone). Examples of such procedures include biopsies, thermal ablations, the removal of damaged tissue, the insertion of chemical/pharmaceutical products into the human body. The procedure of inserting the surgical/diagnostic instrument and subsequently reaching the target zone may be facilitated by guidance, or navigation systems, of the virtual type, based on images of the target zone and of the areas surrounding the target zone in order to plan and perform the percutaneous procedure in the least invasive way possible.

Known navigation/guidance systems for procedures of the kind described above are based on the processing of images acquired beforehand by means of computerised tomography (CT) or magnetic resonance (MR) and consist in the definition of a virtual three-dimensional reconstruction of the target zone of the human body.

A three-dimensional representation of the surgical/diagnostic instrument is superimposed on the three-dimensional reconstruction and moves over the image following the actual movements of the instrument.

Clearly, to be able to view the position in space of the surgical/diagnostic instrument, this must be provided with appropriate sensors, for instance optical sensors, so that the position of the surgical/diagnostic instrument can be known in real time.

For example, patent EP09425116 filed by this same applicant describes a computer-assisted system for guiding a surgical/diagnostic instrument in the body of a patient comprising a first marker device configured to be arranged so as to be integral with a patient's body region and including first and second patient marker elements having a given reciprocal arrangement; a second marker device configured to be coupled to the surgical/diagnostic instrument and including third instrument marker elements; an optical locating sensor configured to position the second and third marker elements in a first reference system; and a processing unit configured to acquire at least one tomographic image of the patient's body region comprising the first marker elements in a second reference system different from the first, acquire the position of the second and third marker elements in the first reference system and determine the position of said third marker elements in the second reference system on the basis of a correlation between the first and the second reference systems.

The optical sensor generally comprises a pair of cameras suitable to capture an image, from different angles, of an area of vision in which the surgical/diagnostic tool is located.

The optical path between the optical sensor and the patient and instrument marker elements can be interrupted in many operating conditions, for example when medical personnel are positioned in a circle around the patient and obscure the optical marker elements on several sides so that these are not visible to the cameras. WO2017208186 and EP1096268 refer to further prior art documents relevant for the present invention.

### SUBJECT OF THE INVENTION

The purpose of the present invention is to provide a vision system that overcomes the drawbacks of the systems known in the prior art and gives a better view of the optical markers. The present invention is defined by independent claim 1. Preferred embodiments are disclosed in the dependent claims.

The aforesaid purpose is achieved by the present invention in that it relates to a three-dimensional vision system for guiding a surgical/diagnostic instrument in the body of a patient, comprising: at least a first and a second vision device of the optical type that can be arranged in different angular positions around a shared area of surgical intervention in which there is arranged a patient on whom the procedure is to be performed, in order to observe the patient from different viewpoints:
each vision device being provided with a lighting device, in particular an infrared lighting device, and with a pair of cameras with optical axes converging in a shared point and achieving a stereoscopic vision system suitable to identify first optical markers carried by a patient marker which is arranged in a stable and predetermined position on the body of the patient and second optical markers carried by an instrument marker which is carried by an instrument used to perform a diagnostic/surgical procedure on the patient; said first optical markers comprising a plurality of first reflecting members arranged in stable and predetermined positions with respect to one another and said second optical markers comprising a plurality of second reflecting members arranged in stable and predetermined positions with respect to one another; characterised in that it comprises first image processing means which are configured, for each vision system present, to cyclically carry out the following operations on the image detected: detect the presence and the position in space of the first optical markers; detect the presence and the position in space of the second optical markers; if at least one of the first optical markers or second optical markers is not detected, the detection operations are repeated and the respective vision device does not provide any information on the position in space of the patient marker and the instrument marker; if both the first optical markers and the second optical markers are detected, said first image processing means are suitable to store the spatial coordinates of the patient marker and the instrument marker; said first image processing means being suitable to calculate for each respective vision device, a first marker error εₘ that represents the error between the theoretical geometrical position of the first optical markers and the position of the optical markers as detected in the images used to obtain the spatial position of the patient marker; said first image processing means being further suitable to calculate a second instrument error εₛ that represents the error between the theoretical geometrical position of the second optical markers and the position of the optical markers as detected in the images used to obtain the spatial position of the instrument marker;
second image processing means being further provided, that receive, when available, the input coordinates of the instrument marker and the patient marker and the first marker error εₘ and the second instrument error εₛ detected by the different vision systems; said second image processing means are configured to process the data entered, to calculate the overall coordinates **Cg** of the instrument marker that take into account the information processed by the different vision devices operating in parallel; the overall coordinates **Cg** are made available to a virtual navigation system suitable to represent a three-dimensional image of the body of the patient on which a three-dimensional model of the instrument is represented, positioned and orientated based on the overall coordinates **Cg** thus determined.

In this way, the system according to the present invention detects the three-dimensional positions of the optical sensors applied to the surgical/diagnostic instrument in an extremely precise and reliable way; the system is therefore an essential component in guided surgery based on images.

### BRIEF DESCRIPTION OF THE DRAWINGS

The invention will now be described with reference to the accompanying drawings of a preferred non-limiting embodiment thereof in which:
Figure 1 is a perspective view of a three-dimensional vision system for guiding a surgical/diagnostic instrument in the body of a patient produced according to the teachings of the present invention;
Figure 2 is a block diagram of the functioning of the system in Figure 1;
Figure 3 illustrates a patient marker used by the system according to the present invention; and
Figure 4 illustrates an instrument marker used by the system according to the present invention.

### PREFERRED EMBODIMENT OF THE INVENTION

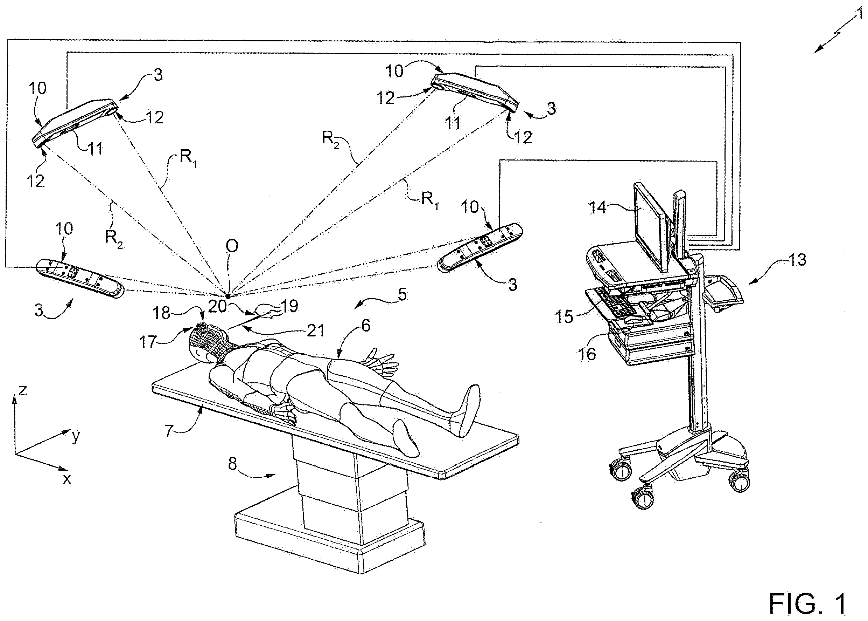

**In** **Figure 1**, reference numeral 1 globally denotes a three-dimensional vision system for guiding a surgical/diagnostic instrument in the body of a patient, produced according to the teachings of the present invention.

The system 1 comprises a plurality of vision devices 3 of the optical type, that can be arranged in different angular positions around a shared area of surgical intervention 5 in which there is arranged a patient 6 on whom a procedure is to be performed, in order to observe the patient 6 from different viewpoints. In the example that is shown, there are four vision devices 3 substantially equally spaced around the area 5; however, it is clear that the number of vision devices 3 may differ from that shown.

In the example that is illustrated, the patient 6 has been placed horizontally on a supporting surface 7 of an operating table 8 around which the medical and paramedical personnel are positioned (not illustrated, for the sake of simplicity) . Each vision device 3 is provided with a head 10 that houses a lighting device 11 (or a pair of lighting devices), in particular an infrared lighting device, and with a pair of cameras 12 with optical axes R1, R2 converging in a point O and achieving a stereoscopic vision system (within a given measurement volume) of a known type and therefore not described in more detail here. Conveniently, the points O of the different vision devices 3 are shared and are arranged at the centre of the shared area of surgical intervention 5. Each vision device cooperates with a shared processing unit 13 which is suitable to identify - using algorithms of the known type - in the images detected by the cameras 12, first optical markers 17 carried by a patient marker 18 (also see

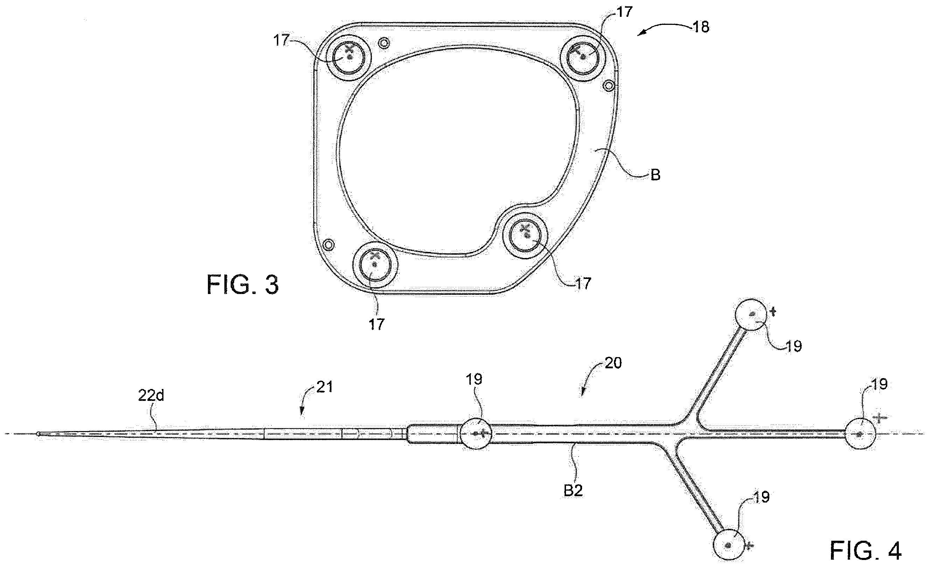

Figure 3) which is arranged in a stable and predetermined position on the body of the patient 6 (for example, resting on the middle of the head as illustrated in the example) and second optical markers 19 carried by an instrument marker 20 (also see Figure 4) which is carried by an instrument 21 used to perform a diagnostic/surgical procedure on the patient 6. For the sake of simplicity, in the example that is illustrated only one instrument 21 provided with a respective instrument marker 20 is described. It is, however, clear that the system can work with a greater number of instruments provided with respective markers.

In the example that is illustrated, the processing unit 13 consists of a personal computer provided with a monitor 14, keyboard 15 and mouse 16.

The first optical markers 17 comprise a plurality of reflecting spherical elements carried by a base B made of plastic material and arranged in stable and predetermined positions with respect to one another. For example, there may be four spherical elements 17 arranged at the vertices of a trapezium.

The second optical markers 19 comprise a plurality of reflecting spherical elements carried by a base B2 and arranged in stable and predetermined positions with respect to one another. For example, there may be four spherical elements 19 arranged at the vertices of a rhombus.

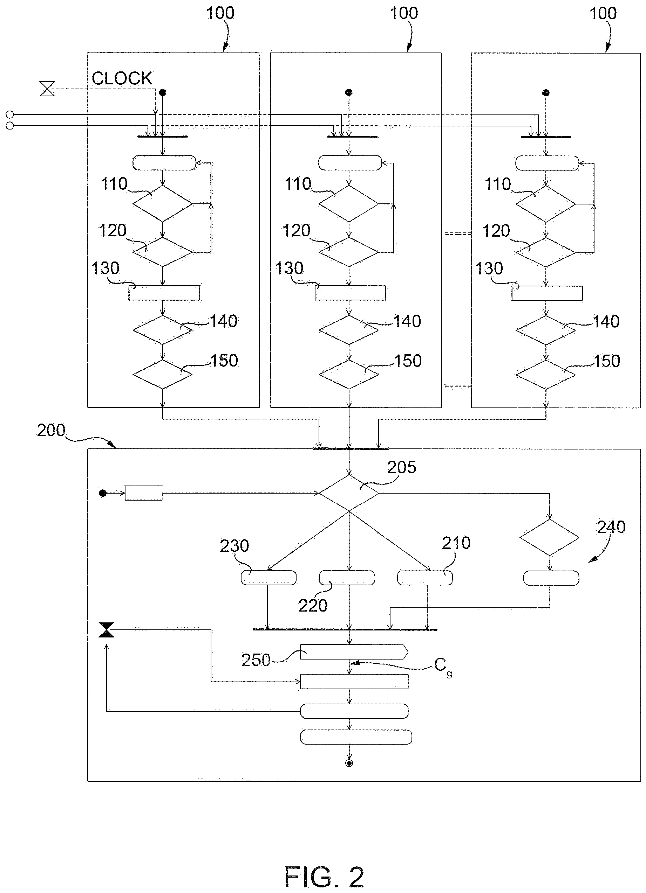

**Figure 2** **illustrates** a logical diagram of the operations performed by the processing unit 13 according to the present invention.

Each rectangular block 100 illustrates a first process related to a respective vision device 3, in the example shown in Figure 2 there are three processes that take place in parallel. Each step of each process 100 is synchronised with a shared time reference (CLOCK) of the processing unit 13. The process 100 comprises a first block 110 in which the presence and the position in space of the first optical markers 17 of the patient 6 are detected in the images provided by the cameras 12. The arrangement of the optical markers is in fact known by the processing unit 13 and image recognition algorithms can be used to search for predetermined shapes in order to find the set of optical markers 17.

When the first optical markers 17 have been detected, the system recognises the presence of the patient marker 18 and the block 110 is followed by a block 120.

The block 120 detects, in the images detected by the cameras 12, the presence and the position in space of the second optical markers 19 of the instrument 21. The arrangement of the optical markers is known by the processing unit 13 and image recognition algorithms can be used to search for predetermined shapes in order to find the set of optical markers 19.

The block 120 is followed by a block 130 that provides the position in space (x,y,z) of the patient marker 18 with respect to a reference system of the vision system (three Cartesian coordinates, Figure 1) and the position in space (x,y,z) and orientation (direction cosines) of the instrument marker 20 as well as the axial rotation. The instrument 20 is in fact provided with a rectilinear portion 22d (Figure 4) that can be held and the direction cosines identify the spatial arrangement of said rectilinear portion 22d.

If at least one of the first optical markers 17 or second optical markers 19 is not detected, the detection operations (blocks 110 and 120) are repeated and the respective vision device 3 does not provide any information on the position in space of the patient marker 18 or the instrument marker 20. If, instead, the blocks 110 and 120 both detect the position of the first optical markers and second optical markers, respectively, the block 120 is followed by a block 130 suitable to store the spatial coordinates of the patient marker 18 and of the instrument marker 20.

The block 130 is followed by a block 140 that is suitable to calculate, for the respective vision device, a first marker error εₘ that represents the error between the theoretical geometrical position of the first optical markers 17 (the theoretical geometrical position is indicated by a dot in Figure 3) and the position of the optical markers as detected in the images (the detected position is indicated by a cross in Figure 3) used to obtain the three-dimensional position of the patient marker 18.

The theoretical position is the coordinate stored in the computer for the sensor model.

It is called theoretical because the coordinates are those of the individual markers as per drawing specifications.

When the patient sensors or the probe are detected, the measured coordinates of the single markers are relativised in distances (4 markers provide 6 distances, 4 sides and 2 diagonals). If these measured distances differ from those "as per drawing specifications" or the theoretical distances, an epsilon error occurs.

Let us assume that our sensor has 4 spherical elements arranged at the vertices of a square with the side 100 as per drawing specifications (or theoretical).
Spherical element 1 coordinates 0,0
Spherical element 2 coordinates 100,0
Spherical element 3 coordinates 0,100
Spherical element 4 coordinates 100,100

We have 6 theoretical distances (1-2:100; 2-3:100; 3-4:100; 4-1:100; 1-3:141; 2-4:141)

When we measure, we may have deviations, for example:
distances measured (1-2:101; 2-3:98; 3-4:100; 4-1:101; 1-3:142; 2-4:143)
deviations: 1; -2; 0; 1; 1; 2
sqrt error (1+4+0+1+1+4) = 3.31

For example, the marker error εₘ can be calculated overall by determining the theoretical geometrical position of the centroid of the optical markers 17 and determining the position of the centroid of the optical markers 17 as shown in the images; the distance (scalar) between the two centroids represents the error.

The block 140 is followed by a block 150 that is suitable to calculate, for the respective vision device, a second instrument error εₛ that represents the error between the theoretical geometrical position of the second optical markers 19 (the theoretical geometrical position is indicated by a dot in Figure 3) and the position of the optical markers as detected in the image used to obtain the three-dimensional position of the instrument 20. Also in this case, the instrument error εₛ can be calculated overall by determining the theoretical geometrical position of the centroid of the optical markers 19 and determining the position of the centroid of the optical markers 19 as shown in the images; the distance (scalar) between the two centroids represents the error.

All the blocks 150 converge in a second shared process 200 that receives, when available, the input spatial coordinates of the patient marker 18 and of the instrument marker 20 and the first marker error εₘ and the second instrument error εₛ detected by each vision device 3.

The second process 200 is suitable to process the input data to calculate the overall coordinates **Cg** (position and orientation) of the instrument marker 20 that take into account the information processed by the different vision devices 3 operating in parallel.

The overall coordinates **Cg** are made available to a virtual navigation system (of a known type) suitable to represent a three-dimensional image of the body of the patient 6 on which a three-dimensional model of the instrument 21 being used is represented, positioned and orientated based on the overall coordinates **Cg** thus determined.

The three-dimensional navigation system can perform a calibration step using different methods, namely:
a) **Method A** - a plurality of two-dimensional CT or MR images (axial sections) of the patient are imported beforehand, these images are reconstructed using an algorithm to produce a three-dimensional image of a region of interest of the patient, the corresponding region of interest of the patient is scanned using a sensor (for example a laser or a contact sensor) to detect curves that define the three-dimensional position of scanned points on the surface of the patient, the scanned curves are compared against corresponding curves extracted on the three-dimensional image to calculate the rototranslational matrix that aligns the reconstructed three-dimensional image with the image detected of the patient. The three-dimensional image repositioned in the reference system of the vision devices is used in the virtual navigation system by superimposing a model of the instrument 21 on this image. This procedure is described in European patent application EP-A-2.799.029; and
b) **Method B** - a plurality of two-dimensional CT or MR images (axial sections) of the patient are acquired which show the markers detectable by the CT or MR arranged in positions corresponding to the optical markers 18 of the patient (for example using concentric spherical elements), these images are reconstructed using an algorithm to produce a three-dimensional image of a region of interest of the patient, the patient is analysed by the vision system 1 which only detects the position of the patient optical markers 18, to calculate the roto-translation matrix that aligns the reconstructed three-dimensional image (in which the markers detected by the CT or MR system are visible) with the image detected of the patient (in which the optical markers are visible). The three-dimensional image repositioned in the reference system of the vision devices is used in the virtual navigation system by superimposing a model of the instrument 21 on this image. This procedure is described in European patent application EP-A-2.233.099.

The operator can choose from a plurality of alternative methods to calculate the overall coordinates **Cg** (block 205), including:
block 210 is suitable to calculate the overall coordinates **Cg** by selecting the coordinates of the patient marker 20 provided by the vision device 3 that presents the minimum value of the second instrument error εₛ.
block 220 is suitable to calculate the overall coordinates **Cg** by calculating a weighted average of the coordinates of the patient marker 20 provided by the various vision devices 3: each weight having a value that is inversely proportional to the respective second instrument error εₛ. In this way, the vision devices with the greatest error are underestimated.
block 230 is suitable to calculate the overall coordinates **Cg** by calculating an arithmetic average of the coordinates of the patient marker 20 provided by the various vision devices 3.

Block 240 is suitable to calculate the overall coordinates **Cg** by selecting the coordinates of the instrument marker 20 provided by the vision device 3 that presents, on a whole, the overall minimum value of the first marker error εₘ and of the second instrument error εₛ. To determine such overall value the first marker error εₘ and the second instrument error εₛ can be added together as absolute values or multiplied.

Depending on the method of calculation used, the overall coordinates **Cg** are available in a block 250 where they are stored. Thus, a value **Cg** is available for each clock signal. Conveniently, the block 250 is followed by a block 260 that filters the values of the overall coordinates **Cg** before providing them to the virtual navigation system. Filtering improves the representation of the position of the instrument model and prevents this from being subject to any "discontinuity".

The system 1 described and illustrated has the advantage of extending the measuring range of each single vision device 3 and of improving the reading and visibility of the instrument marker 20 reducing the risk of any interruption in the line of sight between the vision device and instrument marker to a minimum.

## Claims

1. A three-dimensional vision system for guiding a surgical/diagnostic instrument in the body of a patient comprising:
- at least a first and a second vision device (3) of the optical type that can be arranged in different angular positions around a shared area of surgical intervention (5) in which there is arranged a patient on whom a procedure is to be performed, in order to observe the patient (6) from different viewpoints:
each vision device (3) being provided with a lighting device (11), in particular an infrared lighting device, and with a pair of cameras (12) with optical axes converging in a shared point (O) and achieving a stereoscopic vision system suitable to identify first optical markers (17) carried by a patient marker (18) which is arranged in a stable and predetermined position on the body of the patient (6) and second optical markers (19) carried by an instrument marker (20) which is carried by an instrument (21) used to perform a diagnostic/surgical procedure on the patient;
said first optical markers (17) comprising a plurality of first bodies arranged in stable and predetermined positions with respect to one another and said second optical markers (19) comprising a plurality of second bodies arranged in stable and predetermined positions with respect to one another;
wherein the system **comprises** first image processing means which are configured, for each vision system present, to cyclically perform the following operations on the image detected:
- detect the presence and the position in space (110) of the first optical markers (17);
- detect the presence and the position in space (120) of the second optical markers (19);
if at least one of the first optical markers or second optical markers is not detected, the detection operations (110,120) are repeated and the respective vision device (3) does not provide any information on the position in space of the patient marker (18) and the instrument marker (20);
if both the first optical markers and the second optical markers are detected, said first image processing means are configured to
store the spatial coordinates (130) of the patient marker (18) and the instrument marker (20);
said first image processing means (100) being configured to calculate (140) for each respective vision device (3), a first marker error εₘ that represents the error between the theoretical geometrical position of the first optical markers (17) and the position of the optical markers as detected in the images used to obtain the spatial position of the patient marker (18) in a coordinate system fixed to the patient marker;
said first image processing means (100) being further configured to calculate a second instrument error εₛ that represents the error between the theoretical geometrical position of the second optical markers (19) and the position of the optical markers as detected in the images used to obtain the spatial position of the instrument marker (20) in a coordinate system fixed to the instrument marker;
said theoretical geometrical positions are memorized and include the coordinates of the individual markers as per drawing specifications;
second image processing means being further provided, that receive, when available, the input coordinates of the instrument marker and the patient marker and the first marker error εₘ and second instrument error εₛ detected by the different vision systems;
said second image processing means (200) are configured to process the data entered, to calculate the overall coordinates **Cg** of the instrument marker (20) that take into account the information processed by the different vision devices (3) operating in parallel; the overall coordinates **Cg** are made available to a virtual navigation system suitable to represent a three-dimensional image of the body of the patient (6) on which a three-dimensional model of the instrument (21) is represented, positioned and orientated based on the overall coordinates **Cg** thus determined.

2. The system according to claim 1, wherein said second image processing means (210) are suitable to calculate the overall coordinates **Cg** by selecting the coordinates of the patient marker (20) provided by the vision device (3) that presents the minimum value of the second instrument error εₛ.

3. The system according to claim 1, wherein said second image processing means (220) are suitable to calculate the overall coordinates **Cg** by calculating a weighted average of the coordinates of the patient marker (20) provided by the various vision devices (3); each weight having a value that is inversely proportional to the respective second instrument error εₛ.

4. The system according to claim 1, wherein said second image processing means (230) are suitable to calculate the overall coordinates **Cg** by calculating an arithmetic average of the coordinates of the patient marker (20) provided by the various vision devices (3).

5. The system according to claim 1, wherein said second image processing means (240) are suitable to calculate the overall coordinates **Cg** by selecting the coordinates of the instrument marker (20) provided by the vision device (3) that presents, on a whole, the overall minimum value of the first marker error εₘ and of the second instrument error εₛ.

6. The system according to the preceding claims wherein the second image processing means provide, through successive cycles, a plurality of overall coordinates which are filtered by means of a mobile average algorithm before being provided to the virtual navigation system.

## Patentansprüche

1. Dreidimensionales Vision-System zum Führen eines chirurgischen/diagnostischen Instruments im Körper eines Patienten, mit:
mindestens einem ersten und einem zweiten Sichtgerät (3) des optischen Typs, die in verschiedenen Winkelpositionen um einen gemeinsamen chirurgischen Eingriffsbereich (5) herum angeordnet werden können, in dem sich ein Patient befindet, an dem eine Behandlung ausgeführt werden soll, zum Betrachten des Patienten (6) aus verschiedenen Blickwinkeln,
wobei jedes Sichtgerät (3) eine Beleuchtungseinrichtung (11), insbesondere eine Infrarot-Beleuchtungseinrichtung, und ein Paar Kameras (12) mit optischen Achsen aufweist, die in einen gemeinsamen Punkt (O) konvergieren und ein stereoskopisches Vision-System bilden, das dazu geeignet ist, erste optische Markierungen (17) auf einem Patienten-Markierungselement (18), das in einer stabilen und vorgegebenen Position auf dem Körper des Patienten (6) angeordnet ist, und zweite optische Markierungen (19) auf einem Instrumenten-Markierungselement (20) zu identifizieren, das durch ein Instrument (21) getragen wird, das verwendet wird, um eine diagnostische/chirurgische Behandlung am Patienten auszuführen,
wobei die ersten optischen Markierungen (17) eine Vielzahl von ersten Körpern aufweisen, die in stabilen und vorgegebenen Positionen relativ zueinander angeordnet sind, und die zweiten optischen Markierungen (19) eine Vielzahl von zweiten Körpern aufweisen, die in stabilen und vorgegebenen Positionen relativ zueinander angeordnet sind,
wobei das System eine erste Bildverarbeitungseinrichtung aufweist, die dafür konfiguriert ist, für jedes vorhandene Vision-System zyklisch die folgenden Operationen bezüglich des erfassten Bilds auszuführen:
Erfassen des Vorhandenseins und der räumlichen Position (110) der ersten optischen Markierungen (17);
Erfassen des Vorhandenseins und der räumlichen Position (120) der zweiten optischen Markierungen (19), wobei
wenn mindestens eine unter den ersten optischen Markierungen und den zweiten optischen Markierungen nicht erfasst wird, die Erfassungsoperationen (110, 120) wiederholt werden und das jeweilige Sichtgerät (3) keine Information über die räumliche Position des Patienten-Markierungselements (18) und des Instrumenten-Markierungselements (20) bereitstellt,
wenn sowohl die ersten optischen Markierungen als auch die zweiten optischen Markierungen erfasst werden, die erste Bildverarbeitungseinrichtung dafür konfiguriert ist, die räumlichen Koordinaten (130) des Patienten-Markierungselements (18) und des Instrumenten-Markierungselements (20) zu speichern,
wobei die erste Bildverarbeitungseinrichtung (100) dafür konfiguriert ist, für jedes Sichtgerät (3) einen ersten Markierungsfehler εₘ zu berechnen (140), der den Fehler zwischen der theoretischen geometrischen Position der ersten optischen Markierungen (17) und der Position der optischen Markierungen darstellt, die in den Bildern erfasst werden, die verwendet werden, um die räumliche Position des Patienten-Markierungselements (18) in einem am Patienten-Markierungselement festgelegten Koordinatensystem zu erhalten,
wobei die erste Bildverarbeitungseinrichtung (100) ferner dafür konfiguriert ist, einen zweiten Instrumentenfehler εₛ zu berechnen, der den Fehler zwischen der theoretischen geometrischen Position der zweiten optischen Markierungen (19) und der Position der optischen Markierungen darstellt, die in den Bildern erfasst werden, die verwendet werden, um die räumliche Position des Instrumenten-Markierungselements (20) in einem am Instrumenten-Markierungselement festgelegten Koordinatensystem zu erhalten,
wobei die theoretischen geometrischen Positionen gespeichert sind und die Koordinaten der einzelnen Markierungen gemäß Zeichnungsspezifikationen enthalten,
ferner eine zweite Bildverarbeitungseinrichtung bereitgestellt wird, die, falls verfügbar, die Eingangskoordinaten des Instrumenten-Markierungselements und des Patienten-Markierungselements sowie den ersten Markierungsfehler εₘ und den zweiten Instrumentenfehler εₛ empfängt, die durch die verschiedenen Vision-Systemen erfasst werden,
die zweite Bildverarbeitungseinrichtung (200) dafür konfiguriert ist, die eingegebenen Daten zu verarbeiten, um die Gesamtkoordinaten Cg des Instrumenten-Markierungselements (20) zu berechnen, die die durch die verschiedenen parallel arbeitenden Sichtgeräte (3) verarbeitete Information berücksichtigen,
wobei die Gesamtkoordinaten Cg für ein virtuelles Navigationssystem zur Verfügung gestellt werden, das dazu geeignet ist, ein dreidimensionales Bild des Körpers des Patienten (6) darzustellen, auf dem ein dreidimensionales Modell des Instruments (21) basierend auf den derart bestimmten Gesamtkoordinaten Cg dargestellt, positioniert und ausgerichtet wird.

2. System nach Anspruch 1, wobei die zweite Bildverarbeitungseinrichtung (210) dazu geeignet ist, die Gesamtkoordinaten Cg durch Auswählen der Koordinaten des Patienten-Markierungselements (20) zu berechnen, die durch das Sichtgerät (3) bereitgestellt werden, das den minimalen Wert des zweiten Instrumentenfehlers εₛ aufweist.

3. System nach Anspruch 1, wobei die zweite Bildverarbeitungseinrichtung (220) dazu geeignet ist, die Gesamtkoordinaten Cg durch Berechnen eines gewichteten Mittelwerts der Koordinaten des Patienten-Markierungselements (20) zu berechnen, die durch die verschiedenen Sichtgeräte (3) bereitgestellt werden, wobei jedes Gewicht einen Wert hat, der zu dem jeweiligen zweiten Instrumentenfehler εₛ umgekehrt proportional ist.

4. System nach Anspruch 1, wobei die zweite Bildverarbeitungseinrichtung (230) dazu geeignet ist, die Gesamtkoordinaten Cg durch Berechnen eines arithmetischen Mittelwerts der Koordinaten des Patienten-Markierungselements (20) zu berechnen, die durch die verschiedenen Sichtgeräte (3) bereitgestellt werden.

5. System nach Anspruch 1, wobei die zweite Bildverarbeitungseinrichtung (240) dazu geeignet ist, die Gesamtkoordinaten Cg durch Auswahl der Koordinaten des Instrumenten-Markierungselements (20) zu berechnen, die durch das Sichtgerät (3) bereitgestellt werden, das insgesamt den minimalen Gesamtwert des ersten Markierungsfehlers εₘ und des zweiten Instrumentenfehlers εₛ aufweist.

6. System nach einem der vorangehenden Ansprüche, wobei die zweite Bildverarbeitungseinrichtung über aufeinanderfolgende Zyklen eine Vielzahl von Gesamtkoordinaten bereitstellt, die mittels eines Moving-Average-Algorithmus gefiltert werden, bevor sie dem virtuellen Navigationssystem zur Verfügung gestellt werden.

## Revendications

1. Système de vision tridimensionnelle pour le guidage d'un instrument chirurgical/de diagnostic dans le corps d'un patient comprenant :
- au moins un premier et un second dispositif de vision (3) du type optique qui peuvent être agencés dans différentes positions angulaires autour d'une zone partagée d'intervention chirurgicale (5) dans laquelle est agencé un patient sur lequel une procédure doit être réalisée, afin d'observer le patient (6) depuis différents points de vue :
chaque dispositif de vision (3) étant muni d'un dispositif d'éclairage (11), en particulier d'un dispositif d'éclairage infrarouge, et d'une paire d'appareils de prise de vues (12) ayant des axes optiques convergeant en un point partagé (O) et parvenant à un système de vision stéréoscopique approprié pour identifier des premiers repères optiques (17) portés par un repère de patient (18) qui est agencé dans une position stable et prédéterminée sur le corps du patient (6) et des seconds repères optiques (19) portés par un repère d'instrument (20) qui est porté par un instrument (21) utilisé pour réaliser une procédure de diagnostic/chirurgicale sur le patient ;
lesdits premiers repères optiques (17) comprenant
une pluralité de premiers corps agencés dans des positions stables et prédéterminées les uns par rapport aux autres et lesdits seconds repères optiques (19) comprenant une pluralité de seconds corps agencés dans des positions stables et prédéterminées les uns par rapport aux autres ;
dans lequel le système comprend des premiers moyens de traitement d'image qui sont configurés, pour chaque système de vision présent, pour réaliser de manière cyclique les opérations suivantes sur l'image détectée :
- détecter la présence et la position dans l'espace (110) des premiers repères optiques (17) ;
- détecter la présence et la position dans l'espace (120) des seconds repères optiques (19) ;
si au moins l'un parmi les premiers repères optiques ou les seconds repères optiques n'est pas détecté, les opérations de détection (110, 120) sont répétées et le dispositif de vision (3) respectif ne fournit aucune information sur la position dans l'espace du repère de patient (18) et du repère d'instrument (20) ;
si à la fois les premiers repères optiques et les seconds repères optiques sont détectés, lesdits premiers moyens de traitement d'image sont configurés pour stocker les coordonnées spatiales (130) du repère de patient (18) et du repère d'instrument (20) ;
lesdits premiers moyens de traitement d'image (100) étant configurés pour calculer (140), pour chaque dispositif de vision (3) respectif, une première erreur de repère εₘ qui représente l'erreur entre la position géométrique théorique des premiers repères optiques (17) et la position des repères optiques telle que détectée dans les images utilisées pour obtenir la position spatiale du repère de patient (18) dans un système de coordonnées fixé au repère de patient ;
lesdits premiers moyens de traitement d'image (100) étant en outre configurés pour calculer une seconde erreur d'instrument εₛ qui représente l'erreur entre la position géométrique théorique des seconds repères optiques (19) et la position des repères optiques telle que détectée dans les images utilisées pour obtenir la position spatiale du repère d'instrument (20) dans un système de coordonnées fixé au repère d'instrument ;
lesdites positions géométriques théoriques sont mémorisées et incluent les coordonnées des repères individuels selon les spécifications d'un dessin ;
des seconds moyens de traitement d'image étant en outre fournis, qui reçoivent, lorsqu'elles sont disponibles, les coordonnées d'entrée du repère d'instrument et du repère de patient et la première erreur de marqueur εₘ et la seconde erreur d'instrument εₛ détectées par les différents systèmes de vision ;
lesdits seconds moyens de traitement d'image (200) sont configurés pour traiter les données entrées, pour calculer les coordonnées globales Cg du repère d'instrument (20) qui prennent en compte les informations traitées par les différents dispositifs de vision (3) fonctionnant en parallèle ; les coordonnées globales Cg sont rendues disponibles pour un système de navigation virtuelle approprié pour représenter une image tridimensionnelle du corps du patient (6) sur laquelle un modèle tridimensionnel de l'instrument (21) est représenté, positionné et orienté sur la base des coordonnées globales Cg ainsi déterminées.

2. Système selon la revendication 1, dans lequel lesdits seconds moyens de traitement d'image (210) sont appropriés pour calculer les coordonnées globales Cg par la sélection des coordonnées du repère de patient (20) fournies par le dispositif de vision (3) qui présentent la valeur minimale de la seconde erreur d'instrument εₛ.

3. Système selon la revendication 1, dans lequel lesdits seconds moyens de traitement d'image (220) sont appropriés pour calculer les coordonnées globales Cg par le calcul d'une moyenne pondérée des coordonnées du repère de patient (20) fournies par les divers dispositifs de vision (3) ; chaque pondération ayant une valeur qui est inversement proportionnelle à la seconde erreur d'instrument εₛ respective.

4. Système selon la revendication 1, dans lequel lesdits seconds moyens de traitement d'image (230) sont appropriés pour calculer les coordonnées globales Cg par le calcul d'une moyenne arithmétique des coordonnées du repère de patient (20) fournies par les divers dispositifs de vision (3).

5. Système selon la revendication 1, dans lequel lesdits seconds moyens de traitement d'image (240) sont appropriés pour calculer les coordonnées globales Cg par la sélection des coordonnées du repère d'instrument (20) fournies par le dispositif de vision (3) qui présentent, globalement, la valeur minimale globale de la première erreur de marqueur εₘ et de la seconde erreur d'instrument εₛ.

6. Système selon les revendications précédentes dans lequel les seconds moyens de traitement d'image fournissent, par le biais de cycles successifs, une pluralité de coordonnées globales qui sont filtrées au moyen d'un algorithme de moyenne mobile avant d'être fournies au système de navigation virtuelle.
